# EUROPEAN PATENT SPECIFICATION

(11) **EP 2 295 038 B1**
(45) Date of publication and mention of the grant of the patent: **29.05.2013**
(21) Application number: 09170133.4
(22) Date of filing: 11.09.2009
(51) Int. Cl.: A61K 9/16, A61K 31/4164, A61K 31/4166

(54) **Solid dispersion comprising an anti-HIV agent**
Festdispergierung mit einem Anti-HIV-Wirkstoff
Dispersion solide comprenant un agent anti-VIH

(43) Date of publication of application: 16.03.2011
(73) Proprietor: AiCuris GmbH & Co. KG, 42117 Wuppertal (DE)
(72) Inventor: Verhoeven, Ellen, 9820 Merelbeke (BE); Voorspoels, Jody, 8970 Reningelst (BE); Gonnissen, Yves, 3700 Tongeren (BE); Ruysschaert, Yves, 2350 Vosselaar (BE)
(74) Representative: Kilger, Ute

(56) References cited:
- WO-A-02/100853
- WO-A-2009/115213

## Description

The present invention relates to solid dispersions comprising a compound of formula 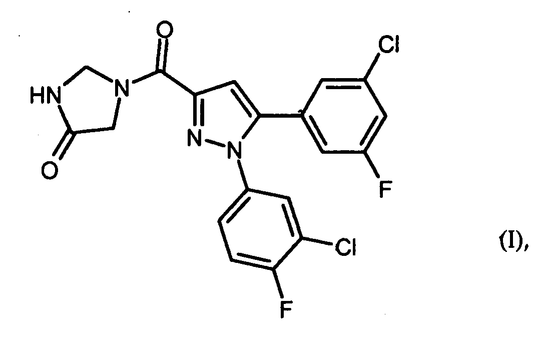 or a salt, a solvate or a solvate of a salt thereof, methods for their production and their use for the production of medicaments as well as to methods for treating and/or preventing viral infections, in particular retroviral infections, and medicaments comprising solid dispersions.

The compound of formula (I) has been developed by the applicant as a promising new candidate for use as a non-nucleosidic reverse transcriptase inhibitor (NNRTI). This compound is expected to proof useful either as a monotherapeutic agent for infections with the human immunodeficiency virus (HIV) or as part of an anti-HIV combination therapy.

HIV causes a chronic persistent progressive infection. The disease proceeds via various stages from the asymptomatic infection to the pathological condition AIDS (*acquired immunodeficiency syndrome*). AIDS is the final stage of the disease caused by the infection. The HIV/AIDS disease is characterized by a long clinical latency period with persistent viraemia which, in the final stage, leads to the failure of the immune defences.

The introduction of the anti-HIV combination therapy made it possible in the 1990s to effectively slow down the progression of the disease and thus to prolong substantially the life expectancy of HIV-infected patients (Palella et al., N. Engl. J. Med. 1998, 238, 853-860).

The anti-HIV substances currently on the market inhibit the replication of the HI virus by inhibiting the essential viral enzymes reverse transcriptase (RT), protease or integrase, or the entry of HIV into the target cell (review in Flexner, Nature Reviews Drug Discovery 2007, 6, 959-966). There are two classes of RT inhibitors: nucleosidic and nucleotidic RT inhibitors (NRTI) act through competitive inhibition or chain termination in the DNA polymerization. Non-nucleosidic RT inhibitors (NNRTI) bind allosterically to a hydrophobic pocket in the vicinity of the active centre of the RT and bring about a conformational change in the enzyme. The currently available protease inhibitors (PI) block the active centre of the viral protease and thus prevent the maturation of newly produced particles into infectious virions. The only currently authorized integrase inhibitor Raltegravir binds in the active centre of the HIV integrase and prevents the integration of the proviral DNA into the host cell genome. Entry inhibitors (fusion inhibitors and coreceptor antagonists) prevent the HIV infection of cells by interacting with the HIV coat protein or by blocking the cellular coreceptors CCR5 or CXCR4.

Since monotherapy with the currently available anti-HIV medicaments leads in a very short time to a failure of the therapy owing to a selection of resistant viruses, usually a combination therapy with several anti-HIV substances from different classes takes place (*highly active antiretroviral therapy* = HAART; Carpenter et al., J. Am. Med. Assoc. 2000, 283, 381-390).

Experiences so far with the compound of formula (I) have shown that while it is highly active against both wild-type and resistant HIV strains this compound tends to form very stable and only sparingly water-soluble crystals. This tendency to form only sparingly water-soluble crystals generally makes it difficult to administer the compound of formula (I) to patients especially via the oral route usually used in HIV therapy and even more difficult to formulate it in such a way that it can be successfully used in combination with other antivirally active agents in an anti-HIV combination therapy.

There is thus a need for novel formulations of the compound of formula (I) which show a distinctly improved dissolution profile in aqueous medium, which are storage-stable and which can be used in combination with other antiretrovirally active agents.

One object of the present invention is therefore to provide formulations of the compound of formula (I) which readily release the compound of formula (I) into an aqueous medium and which show storage stability and in particular a substantially unchanged release profile in aqueous medium even after prolonged storage.

It has surprisingly been found that the solid dispersions of the present invention greatly increase the solubility and dissolution of the compound of formula (I) in an aqueous medium and can be stored over an extended period of time.

The invention relates to solid dispersions comprising a compound of formula 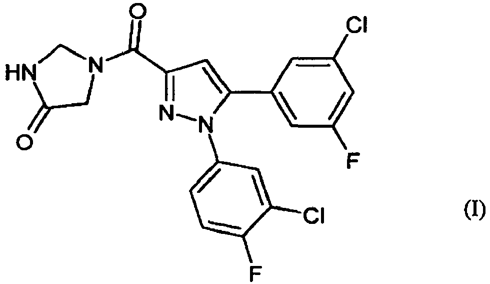 or a salt, a solvate or a solvate of a salt thereof dispersed in a polymeric, inert, non-toxic, pharmaceutically acceptable excipient, wherein the dispersion comprising the compound of formula (I) and the polymeric, inert, non-toxic, pharmaceutically acceptable excipient in a ratio of 1:5 to 1:0.2, preferably 1:2 to 1:0.5 parts by weight.

Whenever in the following description and in the claims reference is made to the compound of formula (I) this also includes the salts, solvates and the solvates of the salts thereof unless explicitly stated otherwise.

Salts for the purposes of the present invention are physiologically acceptable salts of the compound of formula (I).

Physiologically acceptable salts of the compound of formula (I) include acid addition salts of mineral acids, carboxylic acids and sulfonic acids, e.g. salts of hydrochloric acid, hydrobromic acid, sulfuric acid, phosphoric acid, methanesulfonic acid, ethanesulfonic acid, toluenesulfonic acid, benzenesulfonic acid, naphthalenedisulfonic acid, acetic acid, trifluoroacetic acid, propionic acid, lactic acid, tartaric acid, malic acid, citric acid, fumaric acid, maleic acid and benzoic acid.

Physiologically acceptable salts of the compound of formula (I) also include salts of usual bases such as, by way of example and preferably, alkali metal salts (e.g. sodium and potassium salts), alkaline earth metal salts (e.g. calcium and magnesium salts) and ammonium salts derived from ammonia or organic amines having 1 to 16 C atoms, such as, by way of example and preferably, ethylamine, diethylamine, triethylamine, ethyldiisopropylamine, monoethanolamine, diethanolamine, triethanolamine, dicyclohexylamine, dimethylaminoethanol, procaine, dibenzylamine, *N*-methylmorpholine, arginine, lysine, ethylenediamine and *N*-methylpiperidine.

Solvates for the purposes of the invention refer to those forms of the compound of formula (I) which in the solid or liquid state form a complex by coordination with solvent molecules. Hydrates are a specific form of solvates in which the coordination takes place with water.

The expression polymeric, inert, non-toxic, pharmaceutically acceptable excipient includes all solid excipients known to a man of the art having a polymeric structure. Examples for such excipients include artificial polymers such as polymethacrylates, polyvinyl acetates, polyvinyl alcohols, polyvinylpyrrolidones, and derivatives thereof or copolymers including these polymers as well as natural polymers such as celluloses and modified natural polymers such as cellulose derivatives. The expression also includes mixtures of the polymeric excipients.

Is has surprisingly been found that solid dispersions comprising a compound of formula (I) dispersed in a polymeric, inert, non-toxic, pharmaceutically acceptable excipient enhance the solubility and dissolution of the compound of formula (I) and furthermore show enhanced storage stability. In particular, the solid dispersions of the present invention suppress the formation of crystals of the compound of formula (I) which can be observed for example during the storage of amorphous forms of the compound of formula (I) and which strongly decreases the solubility and dissolution of the compound of formula (I).

In a preferred embodiment of the invention the polymeric, inert, non-toxic, pharmaceutically acceptable excipient is selected from the group consisting of polyvinylpyrrolidone, cellulose based polymers and mixtures thereof, in particular from the group consisting of polyvinylpyrrolidone, carboxymethyl cellulose, cellulose acetate, cellulose acetate phthalate, ethyl cellulose, hydroxyethyl cellulose, hydroxyethylmethyl cellulose, hydroxypropyl cellulose, hydroxypropylmethyl cellulose, hydroxypropylmethyl cellulose acetate succinate, hydroxypropylmethyl cellulose phthalate and mixtures thereof and especially from the group consisting of polyvinylpyrrolidone K30, hydroxypropylmethyl cellulose acetate succinate, hydroxypropyl cellulose LF, hydroxypropylmethyl cellulose E5, and hydroxypropylmethyl cellulose phthalate HP-50 and mixtures thereof.

The expression cellulose based polymers includes all excipients known to a man of the art which consist of, are based on or are derived from cellulose, such as various forms of cellulose itself, such as microcrystalline cellulose, powdered cellulose, and silicified microcrystalline cellulose as well as cellulose derivatives, such as carboxymethyl cellulose, cellulose acetate, cellulose acetate phthalate, ethyl cellulose, hydroxyethyl cellulose, hydroxyethylmethyl cellulose, hydroxypropyl cellulose, hydroxypropylmethyl cellulose (also referred to as hypromellose), hydroxypropylmethyl cellulose acetate succinate (also referred to as hypromellose acetate succinate) and hydroxypropylmethyl cellulose phthalate (also referred to as hypromellose phthalate).

It has been shown that solid dispersions of the invention comprising these polymeric, inert, non-toxic, pharmaceutically acceptable excipients show a particularly quick and improved release profile in combination with good storage stability.

In a further embodiment of the present invention the solid dispersion is amorphous.

It has been shown that an amorphous dispersion leads to a particularly good release profile of the compound of formula (I) in aqueous medium.

The invention further relates to a method for producing a solid dispersion of a compound of formula 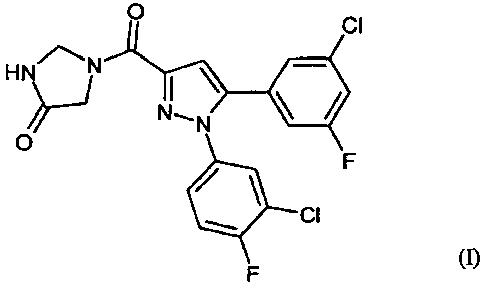 or a salt, a solvate or a solvate of a salt thereof, comprising forming a solution comprising the compound of formula (I) or a salt, a solvate or a solvate of a salt thereof, and a polymeric, inert, non-toxic, pharmaceutically acceptable excipient, dispersing the solution finely and drying the solution, wherein the compound of formula (I) and the polymeric, inert, non-toxic, pharmaceutically acceptable excipient are present in a ratio of 1:5 to 1:0.2, preferably 1:2 to 1:0.5 parts by weight.

According to this method in a first step a solution comprising the compound of formula (I) or a salt, a solvate or a solvate of a salt thereof, and a polymeric, inert, non-toxic, pharmaceutically acceptable excipient in a suitable solvent is formed. This can take place by dissolving both ingredients in one solvent or by forming two individual solutions and mixing these solutions for example shortly before they are finely dispersed. Suitable solvents include any solvent, in which both the compound of formula (I) and the polymeric, inert, non-toxic, pharmaceutically acceptable excipient are soluble. Suitable solvents include generally used and medically acceptable solvents, such as chlorinated hydrocarbons or alcohols or mixtures thereof as well as supercritical fluids, such as for example supercritical CO₂. The solutions in general are prepared such that they contain from 0.1 to 10% by weight of solid, in particular 0.2 to 5 % and especially 0.5 to 1.5% by weight of solid.

The solutions obtained this way are then dispersed finely and dried. This can either take place by direct evaporation of the solvent e.g. in a spray-drying process or the finely dispersed solution can be first deposited onto another carrier and dried there, such as for example in a bit coating technique.

The invention further relates to a method for producing a solid dispersion of a compound of formula 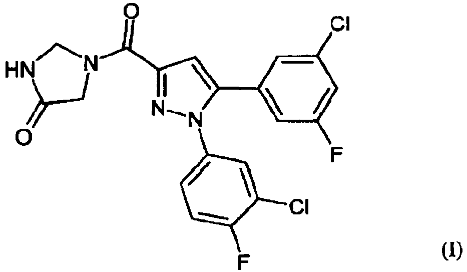 or a salt, a solvate or a solvate of a salt thereof, comprising forming a mixture comprising the molten compound of formula (I) or a salt, a solvate or a solvate of a salt thereof, and a molten, polymeric, inert, non-toxic, pharmaceutically acceptable excipient and extruding the mixture.

This method is generally referred to as a melt extrusion method. According to this method an intimate mixture of the molten compound of formula (I) or a salt, a solvate or a solvate of a salt thereof and the molten, polymeric, inert, non-toxic, pharmaceutically acceptable excipient is formed. This can take place by either mixing the compound of formula (I) or a salt, a solvate or a solvate of a salt thereof, and the polymeric, inert, non-toxic, pharmaceutically acceptable excipient in the solid state and then melting this mixture, or by melting either one or both of the compound of formula (I) or a salt, a solvate or a solvate of a salt thereof and the polymeric, inert, non-toxic, pharmaceutically acceptable excipient and then mixing the ingredients together as long as in the end an intimate molten mixture of both types of ingredients is formed. This mixture is then extruded according to methods known to a man of the art whereby upon cooling of the mixture solidification occurs and a solid dispersion of the compound of formula (I) or a salt, a solvate or a solvate of a salt thereof in the polymeric, inert, non-toxic, pharmaceutically acceptable excipient is formed.

The compound of formula (I) can be synthesized by reacting a compound of formula 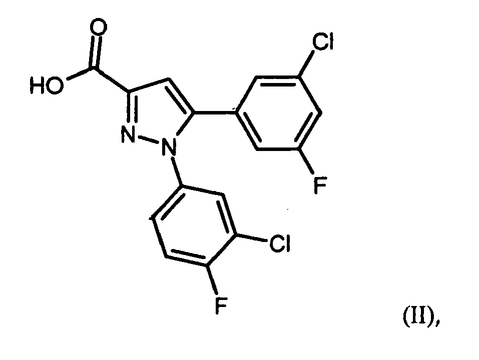 with imidazolidin-4-one or a salt of imidazolidin-4-one.

The reaction generally takes place in inert solvents in the presence of a dehydrating reagent, where appropriate in the presence of a base, preferably in a temperature range from -30°C to 50°C under atmospheric pressure.

Examples of inert solvents are halohydrocarbons such as dichloromethane or trichloromethane, hydrocarbons such as benzene or toluene, nitromethane, tetrahydrofuran, 1,4-dioxane, dimethylformamide or acetonitrile. It is likewise possible to employ mixtures of the solvents. Dichloromethane, dimethylformamide, tetrahydrofuran or toluene are particularly preferred.

Examples of bases are alkali metal carbonates such as, for example, sodium or potassium carbonate, or bicarbonate, or organic bases such as trialkylamines, e.g. triethylamine, *N*-methylmorpholine, *N*-methylpiperidine, 4-dimethylaminopyridine or diisopropylethylamine.

Examples of suitable dehydrating reagents in this connection are carbodiimides such as, for example, *N*,*N*'-diethyl-, *N*,*N*'-dipropyl-, *N*,*N*'-diisopropyl-, *N,N'-*dicyclohexylcarbodiimide, *N*-(3-dimethylaminoisopropyl)-*N*'-ethylcarbodiimide hydrochloride (EDC), *N*-cyclohexylcarbodiimide-N'-propyloxymethylpolystyrene (PS-carbodiimide) or carbonyl compounds such as carbonyldiimidazole, or 1,2-oxazolium compounds such as 2-ethyl-5-phenyl-1,2-oxazolium 3-sulfate or 2-*tert*-butyl-5-methylisoxazolium perchlorate, or acylamino compounds such as 2-ethoxy-1-ethoxycarbonyl-1,2-dihydroquinoline, or propanephosphonic anhydride, or isobutyl chloroformate, or bis(2-oxo-3-oxazolidinyl)phosphoryl chloride, or *O*-(benzotriazol-1-yl)-*N,N,N',N'*-tetramethyluronium hexafluorophosphate (HBTU), 2-(2-oxo-1-(2H)-pyridyl)-1,1,3,3-tetramethyluronium tetrafluoroborate (TPTU) or *O-*(7-azabenzotriazol-1-yl)-*N,N,N',N'*-tetramethyluronium hexafluorophosphate (HATU), or 1-hydroxybenzotriazole (HOBt), or benzotriazol-1-yloxytris(dimethylamino)phosphonium hexafluorophosphate (BOP), or benzotriazol-1-yloxytris(pyrrolidino)phosphonium hexafluorophosphate (PyBOP), or *N*-hydroxysuccinimide, or mixtures thereof, with bases.

The condensation is preferably carried out with PyBOP, TBTU or with EDC in the presence of HOBt.

In an alternative method, the compound of formula (II) can initially be reacted with thionyl chloride and in the second stage with imidazolidin-4-one or a salt of imidazolidin-4-one in the presence of a base such as, for example, triethylamine.

The compound of formula (II) is known or can be prepared by hydrolyzing the ester in a compound of formula 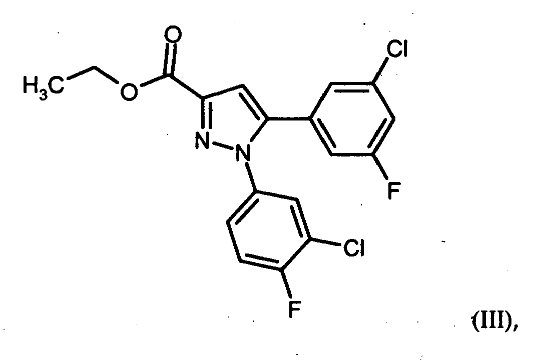 with a base.

The hydrolysis of the ester with a base generally takes place in inert solvents, preferably in a temperature range from room temperature to the reflux of the solvent under atmospheric pressure.

Examples of bases are alkali metal hydroxides such as sodium, lithium or potassium hydroxide, or alkali metal carbonates such as caesium, sodium or potassium carbonate, with preference for lithium, potassium or sodium hydroxide.

Examples of inert solvents are halohydrocarbons such as methylene chloride, trichloromethane, tetrachloromethane, trichloroethane, tetrachloroethane, 1,2-dichloroethane or trichloroethylene, ethers such as diethyl ether, methyl *tert*-butyl ether, 1,2-dimethoxyethane, 1,4-dioxane, tetrahydrofuran, glycol dimethyl ether or diethylene glycol dimethyl ether, alcohols such as methanol, ethanol, n-propanol, isopropanol, n-butanol or *tert*-butanol, hydrocarbons such as benzene, xylene, toluene, hexane, cyclohexane or petroleum fractions, or other solvents such as dimethylformamide, dimethylacetamide, dimethyl sulfoxide, acetonitrile or pyridine, or water, or mixtures of solvents. Preferred solvents are 1,4-dioxane, tetrahydrofuran and/or methanol. Lithium hydroxide in tetrahydrofuran- or 1,4-dioxane-water mixtures or potassium hydroxide in methanol is preferred.

The compound of formula (III) is known or can be prepared by reacting in the first stage a compound of formula 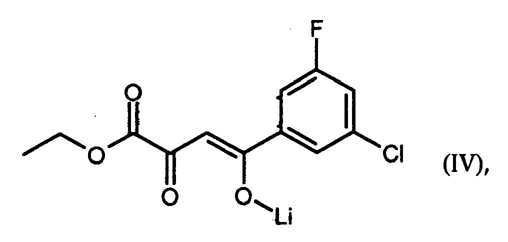 with a compound of formula 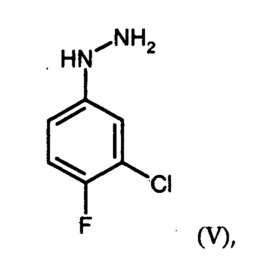 or a salt of the compound of formula (V), and in the second stage heating in acetic acid.

The reaction in the first stage generally takes place in inert solvents, preferably in a temperature range from room temperature to the reflux of the solvent under atmospheric pressure.

Examples of inert solvents are alcohols such as methanol, ethanol, n-propanol, isopropanol, n-butanol, *tert*-butanol or 2-methoxyethanol, with preference for ethanol.

The reaction of the second stage in acetic acid generally takes place in a temperature range from room temperature to the reflux of the acetic acid under atmospheric pressure. The reaction can also be carried out in methanol, ethanol or dioxane in a temperature range from room temperature to the reflux of the solvents. Mixtures of methanol, ethanol or dioxane with acetic acid in the ratio from 0.5/99.5 to 99.5/0.5 by volume are suitable. It is also possible to employ mixtures of methanol, ethanol, dioxane or acetic acid with other acids such as, for example, hydrochloric acid, methanesulfonic acid, p-toluenesulfonic acid, camphorsulfonic acid or trifluoroacetic acid under the conditions mentioned. The reaction is preferably carried out in acetic acid under reflux.

The compounds of formulae (IV) and (V) are known or can be synthesized by known methods from the appropriate precursors.

The preparation of the compound of formula (I) can be illustrated by way of example by the following synthesis scheme.

### Synthesis scheme:

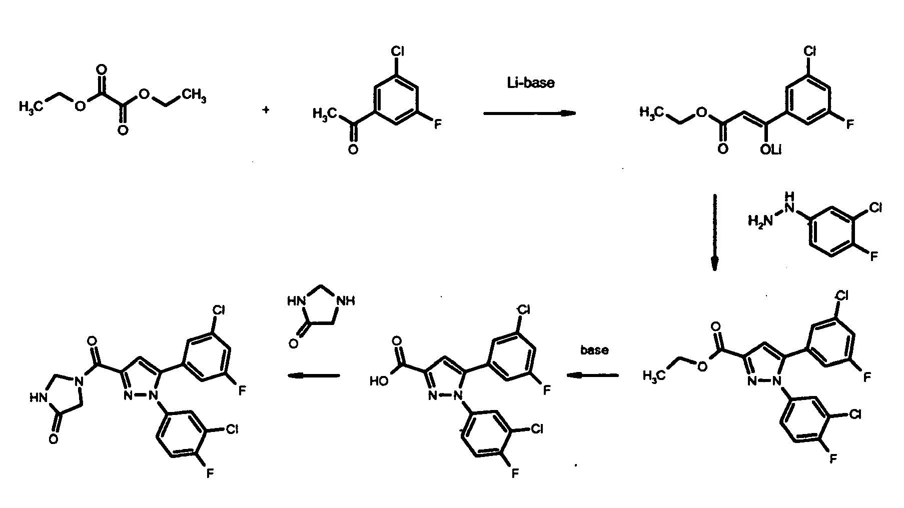

The present invention further relates to the use of the solid dispersions of the invention for the treatment and/or prophylaxis of diseases caused by retroviruses, especially HI viruses.

The present invention further relates to the use of the solid dispersions of the invention for the treatment and/or prophylaxis of diseases, especially of the mentioned diseases.

The present invention further relates to the use of the solid dispersions of the invention for the manufacture of a medicament for the treatment and/or prophylaxis of diseases, especially of the mentioned diseases.

The present invention further relates to a method for the treatment and/or prophylaxis of diseases, especially of the mentioned diseases, using a therapeutically effective amount of the solid dispersions of the invention.

Examples of areas of indication in human medicine which may be mentioned are:
1.) The treatment and prophylaxis of human retroviral infections
2.) The treatment and prophylaxis of infections and diseases (AIDS) caused by HIV-1 (human immunodeficiency virus; formerly called HTLV III / LAV) and HIV-2 and the stages associated therewith, such as ARC (AIDS related complex) and LAS (lymphadenopathy syndrome), as well as the immunodeficiency and encephalopathy caused by this virus.
3.) The treatment of HIV infections caused by mono-, poly- or multiresistant HI viruses.

The expression resistant HI viruses means for example viruses with resistances to nucleosidic inhibitors (NRTI), non-nucleosidic inhibitors (NNRTI) or protease inhibitors (PI) or viruses with resistances to other principles of action, e.g. T20 (fusion inhibitors).
4.) The treatment or prophylaxis of the AIDS-carrier state.
5.) The treatment or prophylaxis of an HTLV-I or HTLV-II infection.

Examples of indications in veterinary medicine which may be mentioned are:
Infections with
   a) Maedi-visna (in sheep and goats)
   b) progressive pneumonia virus (PPV) (in sheep and goats)
   c) caprine arthritis encephalitis virus (in sheep and goats)
   d) zwoegerziekte virus (in sheep)
   e) infectious anaemia virus (of horses)
   f) infections caused by the feline leukaemia virus
   g) infections caused by the feline immunodeficiency virus (FIV)
   h) infections caused by the simian immunodeficiency virus (SIV)

Preference is given from the area of indications in human medicine to items 2, 3 and 4 detailed above.

The solid dispersions of the invention are particularly suitable for controlling HI viruses showing resistances to known non-nucleosidic inhibitors of the reverse transcriptase, such as, for example, etravirine, rilpivirine, efavirenz or nevirapine.

The present invention further relates to medicaments comprising a solid dispersion of the invention and at least one or more further active ingredients, in particular for the treatment and/or prophylaxis of the aforementioned diseases.

The solid dispersions of the invention can also, especially in items 2, 3 and 4 detailed above, advantageously be employed as components of a combination therapy with one or more other compounds which are active in these areas of application. These compounds can for example be employed in combination with effective doses of substances having antiviral activity based on the principles of action detailed below:
HIV protease inhibitors; examples which may be mentioned are: saquinavir, indinavir, ritonavir, nelfinavir, amprenavir, lopinavir, atazanavir, fosamprenavir, tipranavir, darunavir;
nucleosidic, nucleotidic and non-nucleosidic inhibitors of the HIV reverse transcriptase; examples which may be mentioned are: zidovudine, lamivudine, didanosine, zalcitabine, stavudine, lamivudine, abacavir, tenofovir, adefovir, emtricitabine, amdoxovir, apricitabine, racivir, nevirapine, delavirdine, efavirenz, etravirine, rilpivirine, UK-453,061;
HIV integrase inhibitors, examples which may be mentioned are: raltegravir, elvitegravir;
HIV fusion inhibitors; an example which may be mentioned is: enfuvirtide;
Inhibitors of the CXCR4/CCR5/gp120 interaction; examples which may be mentioned are: maraviroc, vicriviroc, INCB009471, AMD-070;
Inhibitors of the polyprotein maturation; an example which may be mentioned is: bevirimat.

This selection is intended to serve to illustrate the possible combinations but not to restrict to the examples detailed here. In principle, every combination of the solid dispersions of the invention with substances having antiviral activity is to be considered as within the scope of the invention.

The present invention further relates to medicaments which comprise a solid dispersion of the invention usually together with at least one further inert, non-toxic, pharmaceutically acceptable excipient as well as to their use for the aforementioned purposes.

It is generally proven advantageous in both human and veterinary medicine to administer the solid dispersion of the invention in such amounts that total amounts from 0.1 to 200 mg/kg, preferably 1 to 200 mg/kg of bodyweight of the compound of formula (I) are administered every 24 hours. In the case of an administration of larger doses these can be administered in the form of a plurality of single doses distributed over the day. A single dose preferably comprises the compound of formula (I) in amounts from 1 to 80 mg/kg, in particular 1 to 30 mg/kg of bodyweight.

It may nevertheless be necessary where appropriate to deviate from the stated amounts in particular as a function of bodyweight, administration route, individual response to the ingredient, type of preparation and time or interval over which the administration takes place. Thus, in some cases it may be sufficient to make due with less than the afore-mentioned minimum amount, whereas in other cases the upper limit mentioned must be exceeded. In the case of an administration of larger amounts it might be advisable to distribute these in a plurality of single doses over the day.

The percentage data in the following tests and examples are unless otherwise indicated percentages by weight, parts are parts by weight. Solvent dilution ratios and concentration data of liquid solutions are based in each case on volume. The statement "w/v" means "weight/volume". Thus, for example, "10% w/v" means 100 ml of solution or suspension contain 10 g of substance.

The invention is hereinafter further described by ways of exemplary embodiments in conjunction with the enclosed drawings in which
Fig. 1 shows a comparison of the X-ray diffraction patterns of a physical mixture of crystalline compound of formula (I) and hydroxypropylmethyl cellulose (HPMC) E5 and of a solid dispersion of the compound of formula (I) in HPMC E5,
Fig. 2 shows a comparison of the X-ray diffraction patterns of a physical mixture of crystalline compound of formula (I) and hydroxypropyl cellulose (HPC) LF and of a solid dispersion of the compound of formula (I) in HPC LF,
Fig. 3 shows a comparison of the X-ray diffraction patterns of a physical mixture of crystalline compound of formula (I) and hydroxypropylmethyl cellulose phthalate (HPMCP) HP-50 and of a solid dispersion of the compound of formula (I) in HPMCP HP-50,
Fig. 4 shows a comparison of the X-ray diffraction patterns of a physical mixture of crystalline compound of formula (I) and polyvinylpyrrolidone (PVP) K30 and of a solid dispersion of the compound of formula (I) in PVP K30,
Fig. 5 shows a comparison of the X-ray diffraction patterns of a freshly made solid dispersion of the compound of formula (I) in HPMC E5 and of the same dispersion after storing at 25°C, 60% relative humidity for two weeks and after storing at 40°C, 75% relative humidity for two weeks,
Fig. 6 shows a comparison of the X-ray diffraction patterns of a freshly made solid dispersion of the compound of formula (I) in HPC LF and of the same dispersion after storing at 25°C, 60% relative humidity for two weeks and after storing at 40°C, 75% relative humidity for two weeks,
Fig. 7 shows a comparison of the X-ray diffraction patterns of a freshly made solid dispersion of the compound of formula (I) in HPMCP HP-50 and of the same dispersion after storing at 25°C, 60% relative humidity for two weeks and after storing at 40°C, 75% relative humidity for two weeks,
Fig. 8 shows a comparison of the X-ray diffraction patterns of a freshly made solid dispersion of the compound of formula (I) in PVP K30 and of the same dispersion after storing at 25°C, 60% relative humidity for two weeks and after storing at 40°C, 75% relative humidity for two weeks,

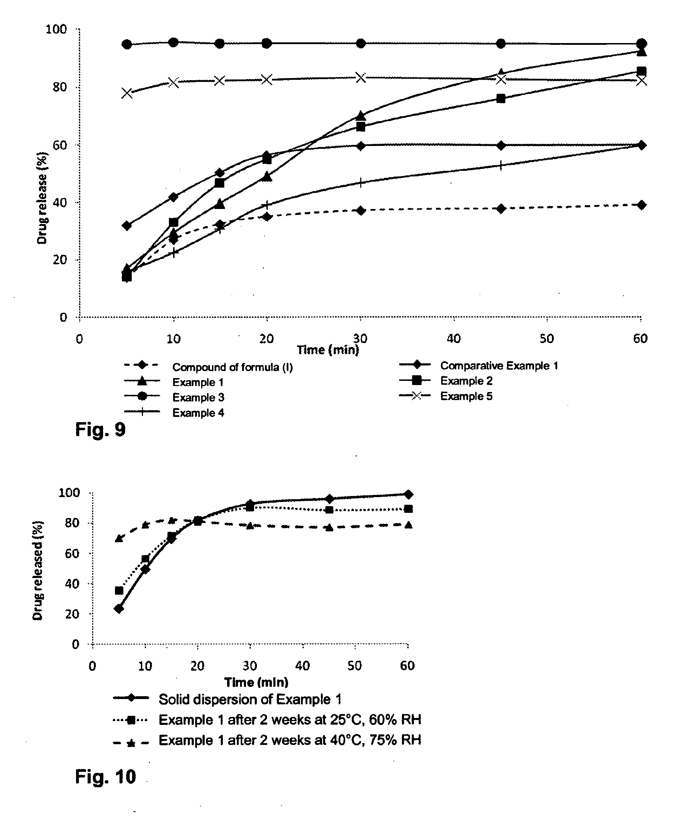

Fig. 9 shows a comparison of the release of the compound of formula (I) from solid dispersions in polymeric excipients compared to the crystalline form of the compound of formula (I) and spray-dried (amorphous) compound of formula (I),
Fig. 10 shows the release profiles of solid dispersions of the compound of formula (I) in HPMC E5 for a freshly made solid dispersion and for the same dispersions after storing at 25°C, 60% relative humidity for two weeks and after storing at 40°C, 75% relative humidity for two weeks,

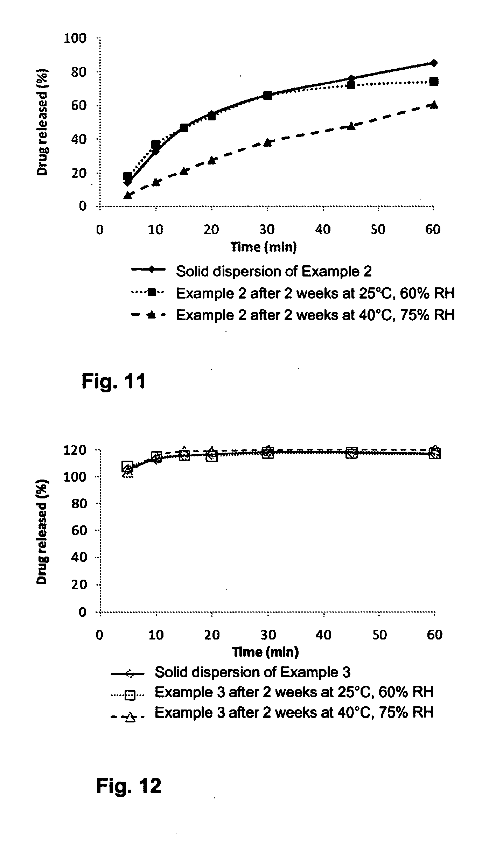

Fig. 11 shows the release profiles of solid dispersions of the compound of formula (I) in HPC LF for a freshly made solid dispersion and for the same dispersion after storing at 25°C, 60% relative humidity for two weeks and after storing at 40°C, 75% relative humidity for two weeks,
Fig. 12 shows the release profiles of solid dispersions of the compound of formula (I) in HPMCP HP-50 for a freshly made solid dispersion and for the same dispersion after storing at 25°C, 60% relative humidity for two weeks and after storing at 40°C, 75% relative humidity for two weeks.

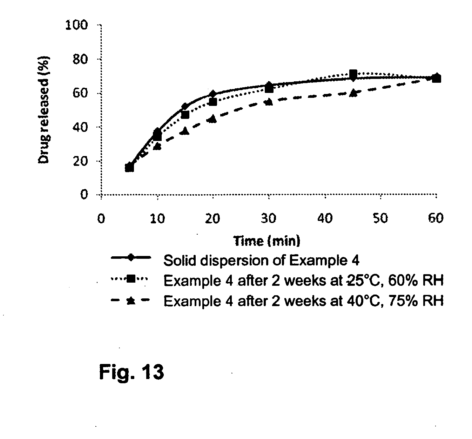

Fig. 13 shows the release profiles of solid dispersions of the compound of formula (I) in PVP K30 for a freshly made solid dispersion and for the same dispersion after storing at 25°C, 60% relative humidity for two weeks and after storing at 40°C, 75% relative humidity for two weeks.

### Exemplary embodiments

### Abbreviations:

- DMSO: dimethyl sulfoxide
- ESI: electrospray ionization (in MS)
- HPLC: high pressure, high performance liquid chromatography
- LC-MS: coupled liquid chromatography-mass spectrometry
- MS: mass spectrometry
- NMR: nuclear magnetic resonance spectroscopy
- PyBOP: benzotriazol-1-yloxytris(pyrrolidino)phosphonium hexafluorophosphate
- Rₜ: retention time (in HPLC)

### A. Synthesis of the compound of formula (I)

### LC-MS methods:

### Method 1:

MS instrument type: Micromass ZQ; HPLC instrument type: HP 1100 Series; UV DAD; column: Phenomenex Gemini 3µ 30 mm x 3.00 mm; eluent A: 11 of water + 0.5 ml of 50% formic acid, eluent B: 11 of acetonitrile + 0.5 ml of 50% formic acid; gradient: 0.0 min 90%A → 2.5 min 30%A → 3.0 min 5%A → 4.5 min 5%A; flow rate: 0.0 min 1 ml/min, 2.5 min/3.0 min/4.5 min 2 ml/min; oven: 50°C; UV detection: 210 nm.

### Method 2:

Instrument: Micromass QuattroPremier with Waters UPLC Acquity; column: Thermo Hypersil GOLD 1.9µ 50 mm x 1 mm; eluent A: 11 of water + 0.5 ml of 50% formic acid, eluent B: 11 of acetonitrile + 0.5 ml of 50% formic acid; gradient: 0.0 min 90%A → 0.1 min 90%A → 1.5 min 10%A → 2.2 min 10%A; oven: 50°C; flow rate: 0.33 ml/min; UV detection: 210 nm.

### Method 3:

MS instrument type: Micromass ZQ; HPLC instrument type: Waters Alliance 2795; column: Phenomenex Synergi 2.5 µ MAX-RP 100A Mercury 20 mm x 4 mm; eluent A: 11 of water + 0.5 ml of 50% formic acid, eluent B: 11 of acetonitrile + 0.5 ml of 50% formic acid; gradient: 0.0 min 90%A → 0.1 min 90%A → 3.0 min 5%A → 4.0 min 5%A → 4.01 min 90%A; flow rate: 2 ml/min; oven: 50°C; UV detection: 210 nm.

### Starting compounds and intermediates:

### Example 1A

### Lithium 1-(3-chloro-5-fluorophenyl)-4-ethoxy-3,4-dioxobut-1-en-1-olate

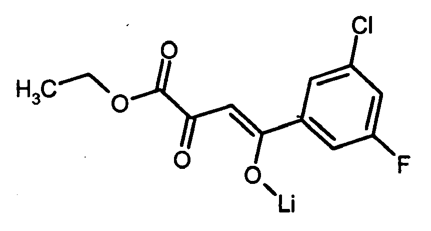

A solution of 78 ml (78 mmol) of lithium hexamethyldisilazide (IN solution in tetrahydrofuran) is provided in 60 ml of diethyl ether at -78°C under argon and a solution of 12.5 g (72.4 mmol) of 1-(3-chloro-5-fluorophenyl)ethanone in 190 ml of diethyl ether is added. After 45 minutes at -78°C, 11.6 g (79.7 mmol) of diethyl oxalate are added dropwise, and the mixture is stirred at room temperature overnight. The reaction mixture is concentrated and 28.6 g of the title compound with 71% purity (100% of theory) are obtained, which are reacted without further purification.

¹H-NMR (400 MHz, DMSO-d₆): δ = 7.63 (t, 1H), 7.55-7.50 (m, 2H), 6.33 (s, 1H), 4.14 (q, 2H), 1.24 (t, 3H).

LC-MS (Method 1): Rₜ = 2.65 min; MS (ESIpos): m/z = 273 [M-Li+2H]⁺.

### Example 2A

### Ethyl 1-(3-chloro-4-fluorophenyl)-5-(3-chloro-5-fluorophenyl)-1H-pyrazole-3-carboxylate

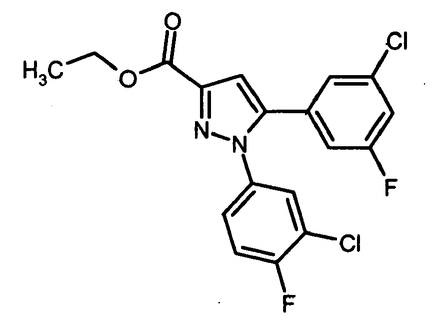

28.6 g of the compound of Example 1A with 71% purity (72.9 mmol) are provided in 350 ml of ethanol, 15.8 g (80.2 mmol) of 3-chloro-4-fluorophenylhydrazine hydrochloride are added, and the mixture is stirred at room temperature overnight. The reaction mixture is concentrated and the residue is taken up in 350 ml of concentrated acetic acid and heated under reflux for 2 h. The reaction mixture is added to ethyl acetate and washed with water and a saturated aqueous sodium bicarbonate solution. The organic phase is concentrated and purified by flash chromatography (mobile phase: cyclohexane/ethyl acetate 20:1). 22.6 g (76% of theory) of the title compound are obtained.

¹H-NMR (400 MHz, DMSO-d₆): δ = 7.80 (dd, 1H), 7.58-7.50 (m, 2H), 7.38 (ddd, 1H), 7.31 (s, 1H), 7.27 (s, 1H), 7.19 (dt, 1H), 4.34 (q, 2H), 1.32 (t, 3H).

LC-MS (Method 2): Rₜ = 1.52 min; MS (ESIpos): m/z = 397 [M+H]⁺.

### Example 3A

### 1-(3-Chloro-4-fluorophenyl)-5-(3-chloro-5-fluorophenyl)-1H-pyrazole-3-carboxylic acid

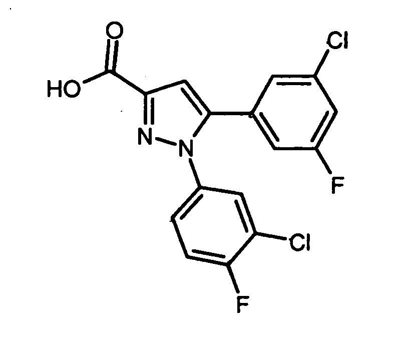

5.11 g (12.9 mmol) of the compound of Example 2A are provided in 142 ml of tetrahydrofuran and, at room temperature, 3.08 g (129 mmol) of lithium hydroxide and 47 ml of water are added. The mixture is stirred at room temperature overnight and, after the addition of a IN aqueous hydrogen chloride solution until the pH is acidic, extracted with ethyl acetate. The organic phase is washed with water, dried over sodium sulfate, filtered and concentrated. 4.51 g (90% of theory) of the title compound are obtained.

¹H-NMR (400 MHz, DMSO-d₆): δ = 13.2 (s, 1H), 7.78 (dd, 1H), 7.58-7.49 (m, 2H), 7.36 (ddd, 1H), 7.28-7.25 (m, 1H), 7.24 (s, 1H), 7.21-7.16 (m, 1H).

LC-MS (Method 1): Rₜ = 2.52 min; MS (ESIpos): m/z = 369 [M+H]⁺.

### Compound of formula (I):

### 1-{[1-(3-Chloro-4-fluorophenyl)-5-(3-chloro-5-fluorophenyl)-1H-pyrazol-3-yl]carbonyl}imidazolidin-4-one

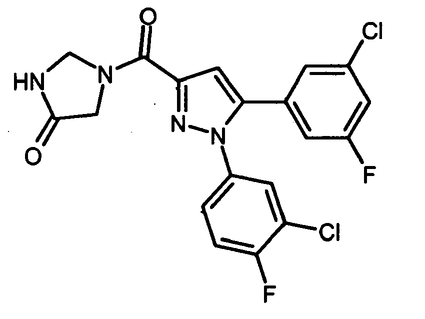

50.0 mg (0.14 mmol) of the compound of Example 3A, 12.8 mg (0.15 mmol) of imidazolidin-4-one and 106 mg (0.20 mmol) of PyBOP are provided in 2.5 ml of tetrahydrofuran and, at room temperature, 50µl (0.28 mmol) of N,N-diisopropylethylamine are added. The reaction mixture is stirred at room temperature overnight and subsequently purified by preparative HPLC (RP18 column; mobile phase: acetonitrile/water gradient). 49.0 mg (83% of theory) of the title compound are obtained.

¹H-NMR (400 MHz, DMSO-d₆): δ = 8.73 (s, 0.6H), 8.63 (s, 0.4H), 7.85-7.81 (m, 1H), 7.58-7.51 (m, 2H), 7.41-7.34 (m, 1H), 7.29-7.26 (m, 2H), 7.22-7.17 (m, 1H), 5.32 (s, 0.8H), 4.90 (s, 1.2H), 4.43 (s, 1.2H), 3.98 (s, 0.8H).

LC-MS (Method 3): Rt = 1.94 min; MS (ESIpos): m/z = 437 [M+H]⁺.

### B. Preparation of the solid dispersions and storage experiments

Organic solutions (methylene chloride/ethanol, ratio: 9/1 w/w) of the compound of formula (I) and the respective polymer (compound of formula (I)/polymer ratio: 1/2 w/w, total solid content: 0.6% w/w, respectively) were prepared. Spray drying of these solutions was performed in a lab scale spray dryer, type B290 inert loop (Buechi, Flawil, Switzerland). The solutions were fed to a two-fluid nozzle (diameter: 0.7 mm) at the top of the spray dryer by means of a peristaltic pump, type 520U (Watson Marlow, Cornwall, UK). The spray dryer operated in co-current air flow. These organic solutions were spray dried according to the process parameters shown in Table 1.

**Table 1: Process conditions during spray drying**

| | |
|---|---|
| Inlet drying nitrogen temperature [°C] | 90 |
| Outlet drying nitrogen temperature [°C] | 50 |
| Condensor temperature [°C] | -20 |
| Drying nitrogen gas rate [m³/h] | 35 |
| Atomising nitrogen pressure [bar] | 0.3 |
| Oxygen concentration [%] | < 6 |

The spray dried particles were collected in a reservoir attached to a cyclone, cooled down to room temperature and stored in sealed vials (room temperature, ambient relative humidity (RH)) prior to their characterisation at To. After characterisation at To, the spray dried solids were stored in open vials for 2 weeks (3 weeks for Comparative Example 1) in desiccators at 25°C, 60% RH and 40°C, 75% RH.

The following examples and comparative examples of solid dispersions and neat compound of formula (I) were prepared:
**Example 1:** Compound of formula (I) and HPMC E5;
**Example 2:** Compound of formula (I) and HPC LF;
**Example 3:** Compound of formula (I) and HPMCP HP-50;
**Example 4:** Compound of formula (I) and PVP K30;
**Example 5:** Compound of formula (I) and hydroxypropylmethyl cellulose acetate succinate (HPMC AS);
**Comparative Example 1:** Spray dried compound of formula (I) without polymer;

The solid dispersions of Example 1 to 5 as well as Comparative Example 1 were storage stable to visual inspection with all dispersions retaining their original white or slightly off-white colouration.

### C. X-ray diffraction

X-ray diffraction (D-500, Siemens, Germany) with CuK_{α} radiation (0.154 nm) was performed on physical mixtures of the compound of formula (I) with HPMC E5, HPC LF, HPMCP HP-50 and PVP K30 and the corresponding solid dispersions (at To and after 2 weeks (T_{2w}) storage at both conditions), as well as spray dried compound of formula (I) without polymer (storage for three weeks under the two conditions). The angular range (2θ) varied from 15 to 60° with steps of 0.02° and the measuring time was 1 s/step.

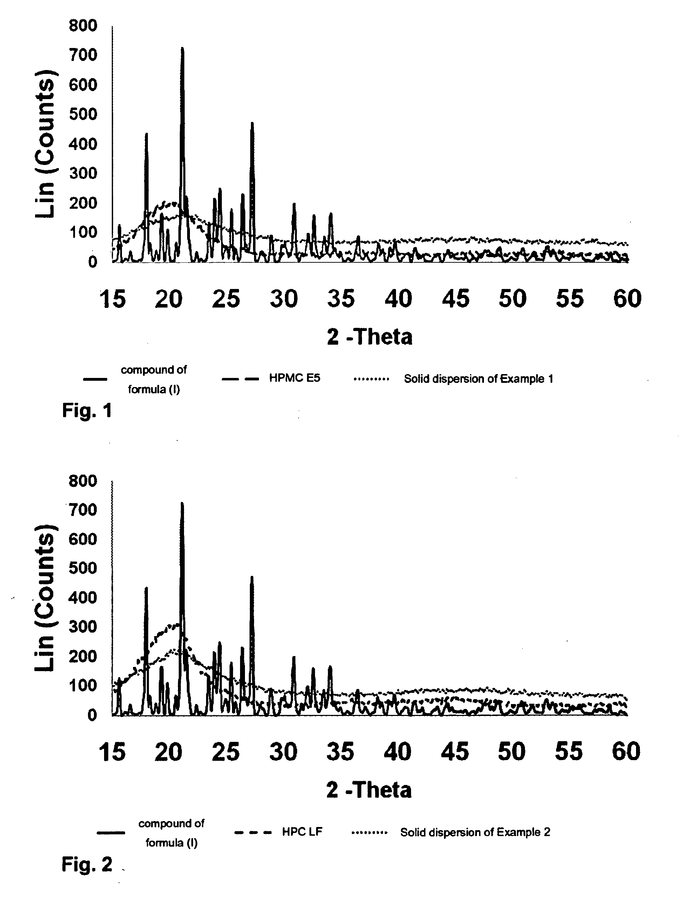

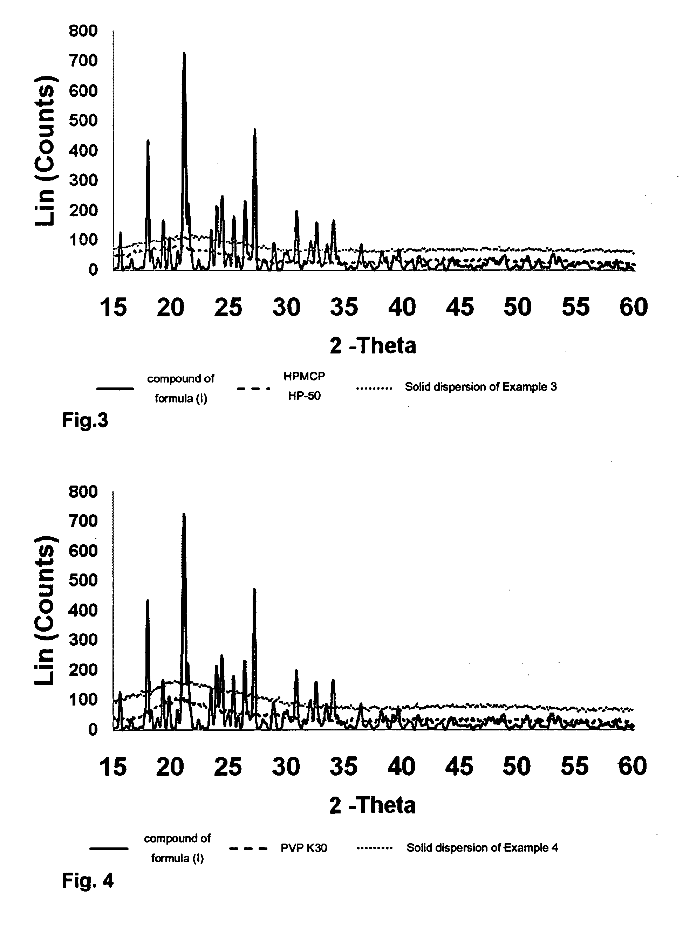

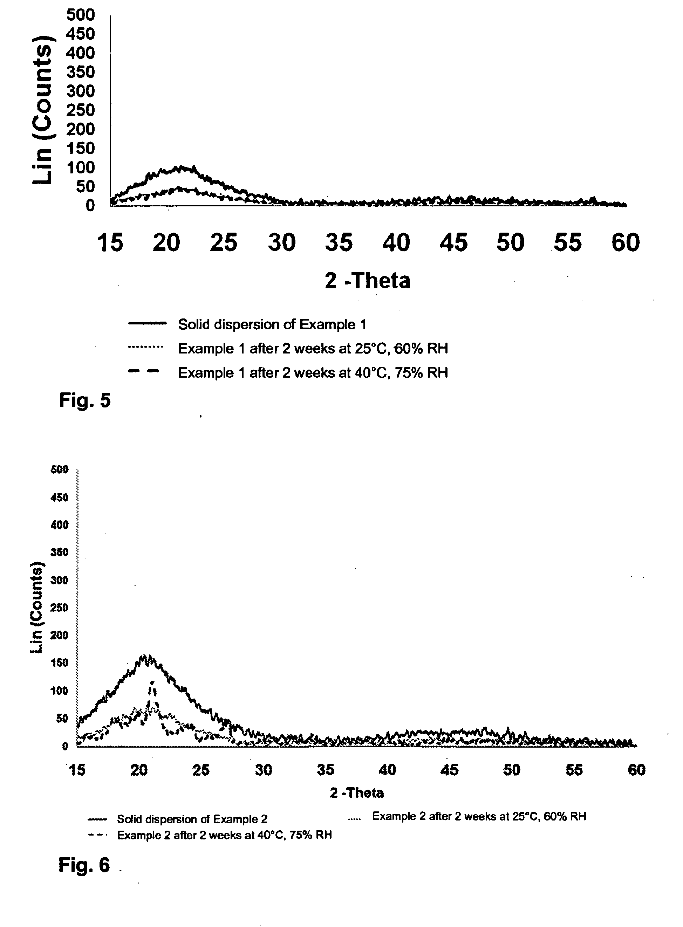

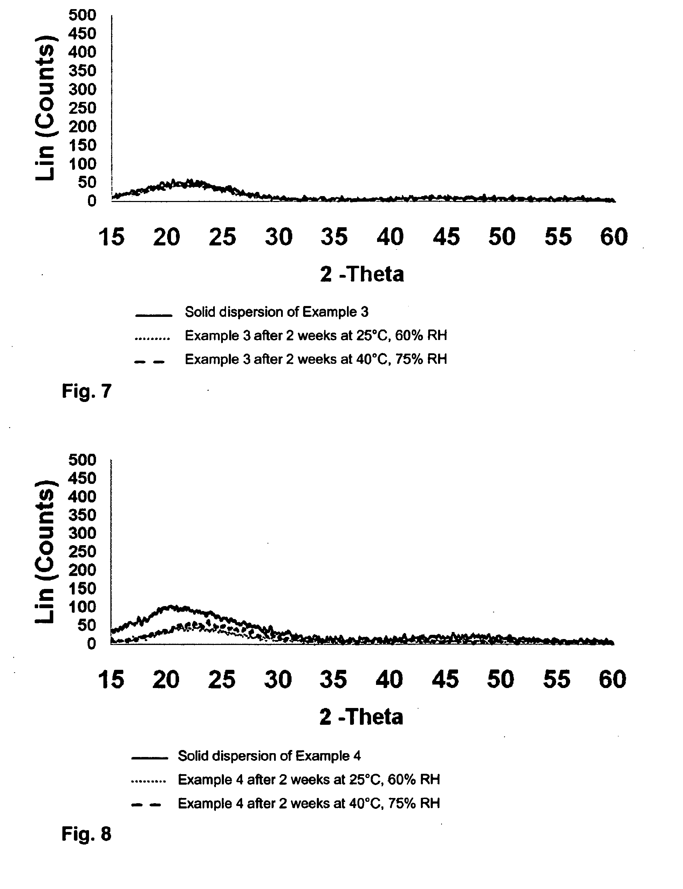

The corresponding spectra for the physical mixtures and the solid dispersions of Examles 1 to 4 are shown in Figs. 1 to 8. The physical mixtures all show the sharp and well defined peaks of the crystalline form of the compound of formula (I). The solid dispersions on the other hand clearly appear as amorphous entities, a fact that is believed to contribute to the enhanced solubility and dissolution of the compound of formula (I) achieved with the solid dispersions of the present invention.

The solid dispersion of Example 2 showed the formation of a small amount of the crystalline form of the compound of formula (I) after two weeks at 40°C, 75% RH but this was judged to be within acceptable limits. All other Examples showed no notable change in the X-ray spectra.

The spray dried form of the compound of formula (I) (Comparative Example 1) on the other hand showed the formation of a large amount of the crystalline form of the compound of formula (I) after 3 weeks at 40°C, 75% RH. The extent of the formation of the crystalline form of the compound of formula (I) was such that this preparation was judged not suitable for long term storage.

### D. Solubility studies

The compound of formula (I) both in crystalline form and spray dried (at To and after 3 weeks (T_{3w}) storage at 25°C, 60% RH and 40°C, 75% RH respectively) as well as the solid dispersions of Examples 1 to 4 (at To and after 2 weeks (T_{2w}) storage at 25°C, 60% RH and 40°C, 75% RH respectively) and Example 5 at To only were introduced in a VK 7000 dissolution system (VanKel, New Jersey, USA), using the paddle method, combined with a VK 8000 automatic sampling station (n = 3). Sodium laurylsulphate 0.5% (w/v) was used as dissolution medium. The temperature of the medium (900 ml) was kept at 37 ± 0.5 °C, while the rotational speed of the paddles was set at 100 rpm. Samples (filtered using Full Flow Filters (Varian) - 35 µm) of 5 ml were withdrawn at 5, 10, 15, 20, 30, 45 and 60 min and spectrophotometrically analysed at 240 nm by UV-spectrophotometry, type UV-1650 (Shimadzu, Deurne, Belgium) (w/w dilution if necessary), using an appropriate calibration curve. Each vessel contained approximately 150 mg of the compound of formula (I).

The compound of formula (I) in its crystalline form showed the expected low solubility with only just about 40% of the compound being dissolved after 60 minutes. The spray dried form of the compound of formula (I) initially shows an improved dissolution with about 60% of the compound being dissolved after 30 minutes. Upon storage at 40°C, 75% RH this effect is rapidly lost though. After storage at these conditions for three weeks only 35% of the compound of formula (I) are dissolved after 60 minutes, making the solubility of this composition worse than that of the fresh crystalline form of the compound of formula (I).

The solid dispersions of the Examples 1 to 5 on the other hand showed a greatly improved solubility for the freshly made dispersions with the dispersion of Example 3 giving the best result with a release of more than 90% of the compound of formula (I) after only 30 minutes. Similar results are obtained with the solid dispersion of Example 1. The solid dispersion of Examples 2 and 5 also gave a useful release of over 80% of the compound of formula (I) after 60 minutes.

Furthermore the solubility of the solid dispersions was only mildly degraded by the storage with the solid dispersions of Examples 3 and 4 virtually unaffected by the storage. The solid dispersions of the Examples 1 and 2 showed a slight reduction of the solubility of the compound of formula (I) through storage, especially under the harsher storage conditions of 40°C, 75% RH (Example 1 going down from 100% release after 60 minutes to about 80% and Example 2 going down from about 80% release to about 60%), but this was judged to be still acceptable for practical applications.

In summary it can be said that the experiments clearly show that solid dispersions of the compound of formula (I) in polymeric excipients lead to formulations having a notably improved solubility and dissolution of the compounds of formula (I). Furthermore these solid dispersion are storage stable and show only little decrease in the solubility and dissolution of the compound of formula (I) after storage.

### E. Exemplary embodiments of pharmaceutical compositions

The compounds of the invention can be converted into pharmaceutical preparations in the following ways:

### Tablet:

### Composition:

500 mg of the solid dispersion of Example 1, 154.4 mg microcrystalline cellulose, 100 mg of lactose (monohydrate), 40 mg of crosscarmellose, 4 mg of magnesium stearate and 1.6 mg silicon dioxide.

Tablet weight 800 mg.

### Production:

The ingredients are mixed thoroughly in a standard mixer and the mixture is compressed using a conventional tablet press. A guideline compressive force for the compression is 15 kN.

## Claims

1. Solid dispersion comprising a compound of formula 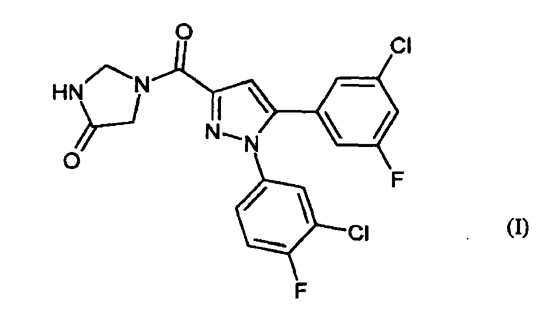 or a salt, a solvate, or a solvate of a salt thereof dispersed in a polymeric, inert, non-toxic, pharmaceutically acceptable excipient **characterized in that** the dispersion comprises the compound of formula (I) and the polymeric, inert, non-toxic, pharmaceutically acceptable excipient in a ratio of 1:5 to 1:0.2, preferably 1:2 to 1:0.5 parts by weight.

2. Solid dispersion according to claim 1, **characterized in that** the polymeric, inert, non-toxic, pharmaceutically acceptable excipient is selected from the group consisting of polyvinylpyrrolidone, cellulose based polymers and mixtures thereof.

3. Solid dispersion according to claim 1 or 2, **characterized in that** the polymeric, inert, non-toxic, pharmaceutically acceptable excipient is selected from the group consisting of polyvinylpyrrolidone, carboxymethyl cellulose, cellulose acetate, cellulose acetate phthalate, ethyl cellulose, hydroxyethyl cellulose, hydroxyethylmethyl cellulose, hydroxypropyl cellulose, hydroxypropylmethyl cellulose, hydroxypropylmethyl cellulose acetate succinate, hydroxypropylmethyl cellulose phthalate and mixtures thereof.

4. Solid dispersion according to any one of claims 1 to 3, **characterized in that** the polymeric, inert, non-toxic, pharmaceutically acceptable excipient is selected from the group consisting of polyvinylpyrrolidone K30, hydroxypropylmethyl cellulose acetate succinate, hydroxypropyl cellulose LF, hydroxypropylmethyl cellulose E5, hydroxypropylmethyl cellulose phthalate HP-50 and mixtures thereof.

5. Solid dispersion according to any one of claims 1 to 4, **characterized in that** it is amorphous.

6. Solid dispersion according to any one of claims 1 to 5 for the treatment and/or prophylaxis of viral infections, preferably of retroviral infections and in particular of infections with the human immunodeficiency virus.

7. Method for producing a solid dispersion of a compound of formula 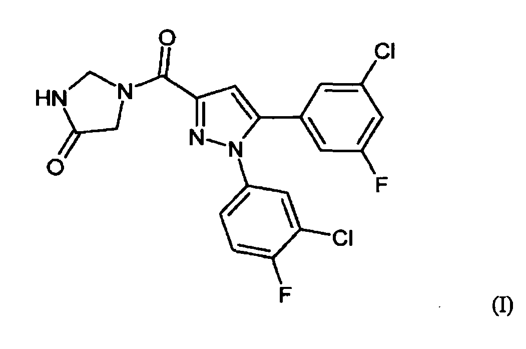 or a salt, a solvate or a solvate of a salt thereof, comprising forming a solution comprising the compound of formula (I) or a salt, a solvate or a solvate of a salt thereof, and a polymeric, inert, non-toxic, pharmaceutically acceptable excipient, dispersing the solution finely and drying the solution, wherein the compound of formula (I) and the polymeric, inert, non-toxic, pharmaceutically acceptable excipient are present in a ratio of 1:5 to 1:0.2, preferably 1:2 to 1:0.5 parts by weight.

8. Method for producing a solid dispersion of a compound of formula 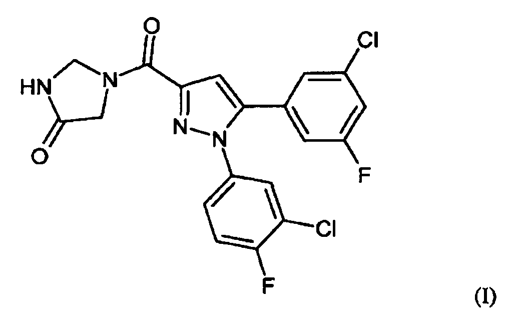 or a salt, a solvate or a solvate of a salt thereof, comprising forming a mixture comprising the molten compound of formula (I) or a salt, a solvate or a solvate of a salt thereof, and a molten, polymeric, inert, non-toxic, pharmaceutically acceptable excipient, and extruding the mixture.

9. Medicament comprising a solid dispersion according to any one of claims 1 to 5 in combination with at least one further active ingredient, preferably an antiretrovirally active ingredient.

10. Medicament comprising a solid dispersion according to any one of claims 1 to 5 in combination with at least one further inert, non-toxic, pharmaceutically acceptable excipient.

11. Medicament according to claim 9 or 10 for the treatment and/or prophylaxis of viral infections, preferably of retroviral infections and in particular of infections with the human immunodeficiency virus.

12. Use of a solid dispersion according to any one of claims 1 to 5, for the production of a medicament for the treatment and/or prophylaxis of viral infections, preferably of retroviral infections and in particular of infections with the human immunodeficiency virus.

13. A solid dispersion according to any one of claims 1 to 5 or of a medicament according to any one of claims 9 to 11 for use in a method for treating and/or preventing viral infections, preferably retroviral infections and in particular infections with the human immunodeficiency virus in humans and animals.

## Patentansprüche

1. Feststoffdispersion, umfassend eine Verbindung mit der Formel 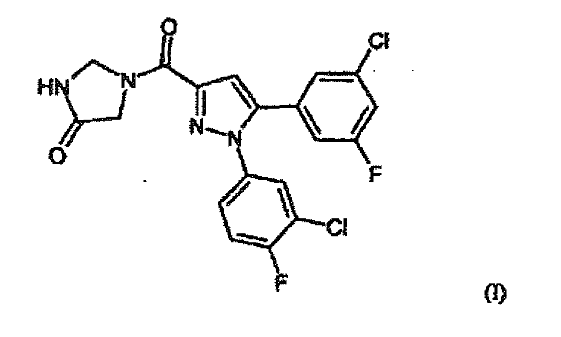 oder ein Salz, ein Solvat, oder ein Solvat eines Salzes davon, welche in einem polymeren, inerten, nicht-toxischen, pharmazeutisch unbedenklichen Hilfsstoff dispergiert ist, **dadurch gekennzeichnet, dass** die Dispersion die Verbindung mit der Formel (I) und dem polymeren, inerten, nicht-toxischen, pharmazeutisch unbedenklichen Hilfsstoff in einem Verhältnis von 1:5 bis 1:0.2, vorzugsweise 1:2 bis 1:0.5 Gewichtsanteile umfasst.

2. Feststoffdispersion nach Anspruch 1, **dadurch gekennzeichnet, dass** der polymere, inerte, nicht-toxische, pharmazeutisch unbedenkliche Hilfsstoff ausgewählt ist aus der Gruppe bestehend aus Polyvinylpyrrolidon, Polymeren auf Zellulosebasis und Gemischen davon.

3. Feststoffdispersion nach Anspruch 1 oder 2, **dadurch gekennzeichnet, dass** der polymere, inerte, nicht-toxische, pharmazeutisch unbedenkliche Hilfsstoff ausgewählt ist aus der Gruppe bestehend aus Polyvinylpyrrolidon, Carboxymethylzellulose, Zelluloseacetat, Zelluloseacetat-Phthalat, Ethylzellulose, Hydroxyethylzellulose, Hydroxyethylmethylzellulose, Hydroxypropylzellulose, Hydroxypropylmethylzellulose, Hydroxypropylmefihylzellulaseacetat-Succinat, Hydroxypropylmethylzellulose-Phthalat und Gemischen davon.

4. Feststoffdispersion nach einem der Ansprüche 1 bis 3, **dadurch gekennzeichnet, dass** der polymere, inerte, nicht-toxische, pharmazeutisch unbedenkliche Hilfsstoff ausgewählt ist aus der Gruppe bestehend aus Polyvinylpyrrolidon K30, Hydroxypropylmethylzellulose-Acetat-Succinat, Hydroxypropylzellulose LF, Hydroxypropylmethylzellulose E5, Hydroxypropylmethylzellulose-Phthalat HP-50 und Gemischen davon.

5. Feststoffdispersion nach einem der Ansprüche 1 bis 4, **dadurch gekennzeichnet, dass** sie amorph ist.

6. Feststoffdispersion nach einem der Ansprüche 1 bis 5 für die Behandlung und/oder Prophylaxe von viralen Infektionen, vorzugsweise von retroviralen Infektionen und insbesondere von Infektionen mit dem humanen Immundefizienz-Virus.

7. Verfahren zur Herstellung einer Feststoffdispersion einer Verbindung mit der Formel 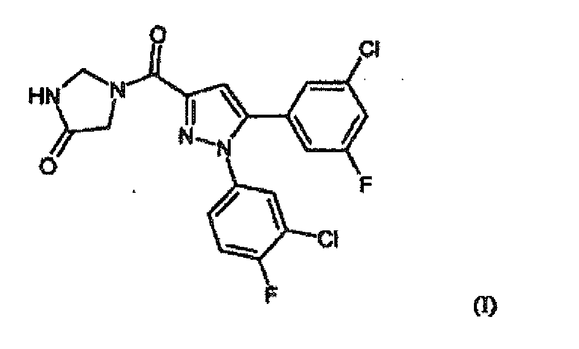 oder ein Salz, ein Solvat oder ein Solvat eines Salzes davon, umfassend die Bildung einer Lösung umfassend die Verbindung mit der Formel (I) oder ein Salz, ein Solvat oder ein Solvat eines Salzes davon, und einen polymeren, inerten, nicht-toxischen, pharmazeutisch unbedenklichen Hilfsstoff, das feine Dispergieren der Lösung und die Trocknung der Lösung, wobei die Verbindung mit der Formel (1) und der polymere, inerte, nicht-toxische, pharmazeutisch unbedenkliche Hilfsstoff in einem Verhältnis von 1:5 bis 1:0.2, vorzugsweise 1:2 bis 1:0.5 Gewichtsanteilen vorhanden sind.

8. Verfahren zur Herstellung einer Feststoffdispersion einer Verbindung mit der Formel 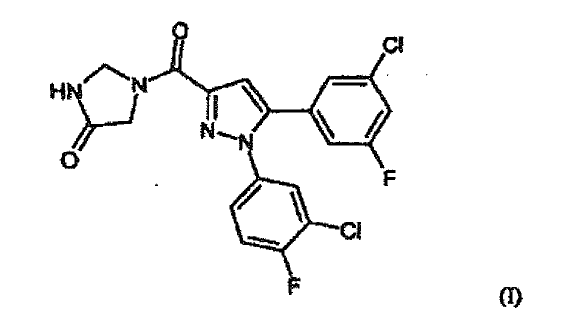 oder ein Salz, ein Solvat oder ein Solvat eines Salzes davon, umfassend die Bildung einer Mischung umfassend die geschmolzene Verbindung mit der Formel (I) oder ein Salz, ein Solvat oder ein Solvat eines Salzes davon, und einen geschmolzenen, polymeren, inerten, nicht-toxischen, pharmazeutisch unbedenklichen Hilfsstoff, und das Extrudieren der Mischung.

9. Medikament umfassend eine Feststoffdispersion nach einem der Ansprüche 1 bis 5 in Verbindung mit mindestens einem weiteren aktiven Inhaltsstoff, vorzugsweise einem antiretroviral aktiven Inhaltsstoff.

10. Medikament umfassend eine Feststoffdispersion nach einem der Ansprüche 1 bis 5 in Verbindung mit mindestens einem weiteren inerten, nicht-toxischen, pharmazeutisch unbedenklichen Hilfsstoff.

11. Medikament nach Anspruch 9 oder 10 für die Behandlung und/oder Prophylaxe von viralen Infektionen, vorzugsweise von retroviralen Infektionen und insbesondere von Infektionen mit dem humanen Immundefizienz-Virus.

12. Verwendung einer Feststoffdispersion nach einem der Ansprüche 1 bis 5 für die Herstellung eines Medikaments für die Behandlung und/oder Prophylaxe von viralen Infektionen, vorzugsweise von retroviralen Infektionen und insbesondere von Infektionen mit dem humanen Immundefizienz-Virus.

13. Feststoffdispersion nach einem der Ansprüche 1 bis 5 oder eines Medikaments nach einem der Ansprüche 9 bis 11 zur Verwendung in einem Verfahren zur Behandlung und/oder Prävention von viralen Infektionen, vorzugsweise retroviralen Infektionen und insbesondere Infektionen mit dem humanen Immundefizienz-Virus bei Menschen und Tieren.

## Revendications

1. Dispersion solide comprenant un composé de la formule 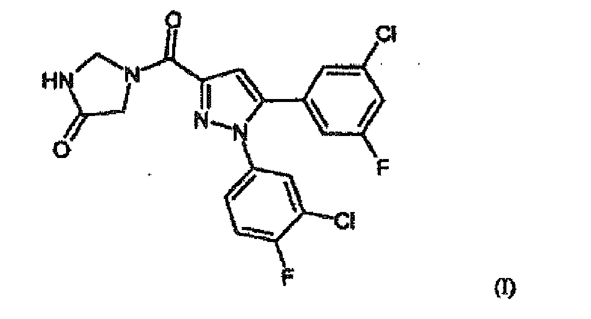 ou un sel, un solvate, ou un solvate d'un sel de ce dernier dispersé dans un excipient polymère, inerte, non-toxique, pharmaceutiquement acceptable, **caractérisé en ce que** la dispersion comprend le composé de la formule (I) et l'excipient polymère, inerte, non-toxique, pharmaceutiquement acceptable dans un rapport de 1:5 à 1:0.2, de préférence 1:2 à 1:0.5 parts par poids.

2. Dispersion solide selon la revendication 1, **caractérisée en ce que** l'excipient polymère, inerte, non-toxique, pharmaceutiquement acceptable est sélectionné dans le groupe constitué par la pyrrolidone de polyvinyle, les polymères à base de cellulose et les mélanges de ces derniers.

3. Dispersion solide selon la revendication 1 ou 2, **caractérisée en ce que** l'excipient polymère, inerte, non-toxique, pharmaceutiquement acceptable est sélectionné dans le groupe constitué par la pyrrolidone de polyvinyle, la carboxyméthylcellulose, l'acétate de cellulose, l'acétate de cellulose phthalate, l'éthylcellulose, l'hydroxyéthylcellulose, l'hydroxyéthylméthylcellulose, l'hydroxypropylcellulose, l'hydroxypropylméthylcellulose, l'acétate-succinate d'hydroxypropylméthylcellulose, le phthalate d'hydroxypropylméthylcellulose et les mélanges de ces derniers.

4. Dispersion solide selon l'une quelconque des revendications 1 à 3, **caractérisée en ce que** l'excipient polymère, inerte, non-toxique, pharmaceutiquement acceptable est sélectionné dans le groupe constitué par la pyrrolidone de polyvinyle K30, l'acétate-succinate d'hydroxypropylméthylcellulose, l'hydroxypropylcellulose LF, l'hydroxypropylméthylcellulose E5, le phthalate d'hydroxypropylméthylcellulose HP-50 et les mélanges de ces derniers.

5. Dispersion solide selon l'une quelconque des revendications 1 à 4, **caractérisée en ce qu'**elle est amorphe.

6. Dispersion solide selon l'une quelconque des revendications 1 à 5 pour le traitement et/ou la prophylaxie d'infections virales, de préférence d'infections rétrovirales et en particulier d'infections par le virus d'immunodéficience humaine.

7. Procédé de production d'une dispersion solide d'un composé de la formule 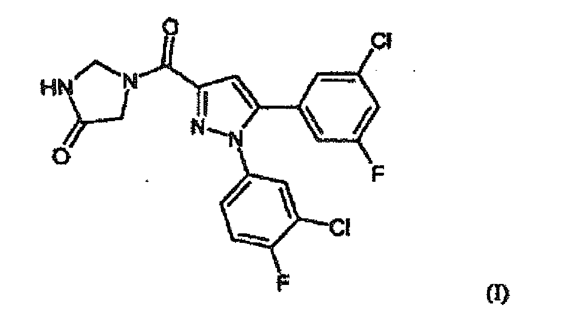 ou d'un sel, un solvate ou un solvate d'un sel de ce dernier comprenant la formation d'une solution comprenant le composé de la formule (I) ou un sel, un solvate ou un solvate d'un sel de ce dernier, et un excipient polymère, inerte, non-toxique, pharmaceutiquement acceptable, la dispersion fine de la solution et séchage de la solution, étant donné que le composé de la formule (1) et l'excipient polymère, inerte, non-toxique, pharmaceutiquement acceptable sont présents dans un rapport de 1:5 à 1:0.2, de préférence 1:2 à 1:0.5 parts par poids.

8. Procédé de production d'une dispersion solide d'un composé de la formule 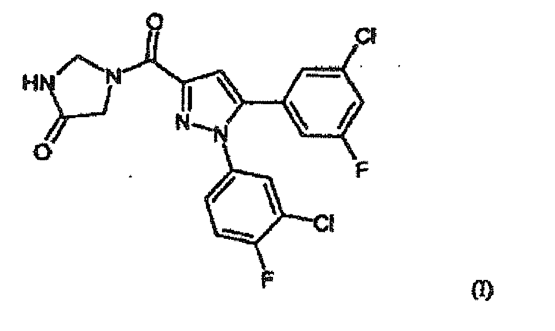 ou un sel, un solvate ou un solvate d'un sel de ce dernier, comprenant la formation d'un mélange comprenant le composé fondu de la formule (I) ou un sel, un solvate ou un solvate d'un sel de ce dernier) et un excipient fondu, polymère, inerte, non-toxique, pharmaceutiquement acceptable, et l'extrusion du mélange.

9. Médicament comprenant une dispersion solide selon l'une quelconque des revendications 1 à 5 en combinaison avec au moins un autre ingrédient actif, de préférence un ingrédient antirétroviralement actif.

10. Médicament comprenant une dispersion solide selon l'une quelconque des revendications 1 à 5 en combinaison avec au moins un autre excipient inerte, non-toxique, pharmaceutiquement acceptable.

11. Médicament selon la revendication 9 ou 10 pour le traitement et/ou la prophylaxie d'infections virales, de préférence d'infections rétrovirales et en particulier d'infections par le virus d'immunodéficience humaine.

12. Utilisation d'une dispersion solide selon l'une quelconque des revendications 1 à 5 pour la production d'un médicament pour le traitement et/ou la prophylaxie d'infections virales, de préférence d'infections rétrovirales et en particulier d'infections par le virus d'immunodéficience humaine.

13. Dispersion solide selon l'une quelconque des revendications 1 à 5 ou d'un médicament selon l'une quelconque des revendications 9 à 11 pour utilisation dans un procédé de traitement et/ou de prévention d'infections virales, de préférence d'infections rétrovirales et en particulier d'infections par le virus d'immunodéficience humaine chez les êtres humains et les animaux.
